# EUROPEAN PATENT APPLICATION

(11) **EP 2 671 859 A1**
(43) Date of publication of application: **11.12.2013**
(21) Application number: 12170640.2
(22) Date of filing: 04.06.2012
(51) Int. Cl.: C07C 5/327, C07C 6/04, C07C 11/02

(54) **Process for the preparation of olefins**

(71) Applicant: Shell Internationale Research Maatschappij B.V., 2596 HR The Hague (NL)
(72) Inventor: Dirkzwager, Hendrik, 1031 HW Amsterdam (NL); Van der Steen, Frederik Hendrik, 1031 HW Amsterdam (NL); Van Westrenen, Jeroen, 1031 HW Amsterdam (NL)
(74) Representative: Matthezing, Robert Maarten

(57) **Abstract**

The invention relates to a process for the preparation of internal monoolefins having of from 20 to 28 carbon atoms, comprising: providing internal monoolefins having of from 10 to 19 carbon atoms; passing a stream comprising the internal monoolefins having of from 10 to 19 carbon atoms to a disproportionation zone; operating the disproportionation zone at disproportionation conditions sufficient to disproportionate monoolefins; and recovering from the disproportionation zone a stream comprising internal monoolefins having of from 20 to 28 carbon atoms.

## Description

### Field of the invention

The present invention relates to a process for the preparation of olefins, in particular a process for the preparation of internal monoolefins having of from 20 to 28 carbon atoms.

### Background of the invention

Heavy internal olefins, for example C20-C28 internal olefins, are needed for the production of internal olefin sulfonates (IOS). Internal olefin sulfonates are useful as surfactants in chemical enhanced oil recovery (cEOR). Compositions and methods for cEOR utilising an IOS as surfactant are described in US4597879, US4979564 and US5068043.

The dehydrogenation of higher paraffins leads to a mixture of olefins which mostly contain internal double bonds. As described in Encyclopedia of Chemical Processing and Design, 1982, vol. 15, pages 266-284, a commercial process for the dehydrogenation of paraffins is UOP's "PACOL" process. However, said process has been directed to preparing lower carbon number olefins, i.e. olefins having of from 10 to 13 carbon atoms, for use in the manufacture of alkyl benzenes as feedstock for detergent manufacture.

EP2186783A2 is directed to the production of higher carbon number olefins for use in the manufacture of surfactants for cEOR applications. In particular, the process of EP2186783A2 comprises dehydrogenating Fischer-Tropsch derived C20-C28 paraffins into C20-C28 monoolefins. Further, EP2186783A2 discloses the separation of said monoolefins from not-dehydrogenated paraffins by an adsorptive separation process. C20-C28 monoolefins may also be obtained via the below-discussed SHOP process, which process however disadvantageously includes an intermediate, double bond isomerization step.

As it is expected that large amounts of IOS surfactants and hence large amounts of heavy internal olefins, for example C20-C28 internal olefins, are required for cEOR, it is desirable if such olefins can also be obtained from other sources. For example, C20-C28 paraffins used as starting material in EP2186783A2 are only available in limited amounts.

In addition, dehydrogenation of paraffins requires a high operating temperature. A negative side-effect of such high operating temperature when dehydrogenating higher carbon number paraffins, such as C20-C28 paraffins, is that more side-reactions occur, such as cracking reactions, rather than the desired dehydrogenation reaction. Because of that, the selectivity disadvantageously decreases.

Another disadvantage in starting from C20-C28 paraffins which have relatively long carbon chains, is the increasing difficulty of separating the desired C20-C28 internal olefins from the starting C20-C28 paraffins. For the longer the carbon chain the more difficult it is to separate an olefin having a certain number of carbon atoms from the corresponding paraffin, because of the relatively small polarity difference between the two.

The objective of the present invention is to provide an alternative process for preparing monoolefins having of from 20 to 28 carbon atoms, which process does not have the above-mentioned drawbacks.

### Summary of the invention

Surprisingly it was found that the above objective is achieved by means of a process wherein first lower carbon number internal monoolefins having of from 10 to 19 carbon atoms are provided, which are then disproportionated resulting in higher carbon number internal monoolefins having of from 20 to 28 carbon atoms.

Accordingly, the present invention relates to a process for the preparation of internal monoolefins having of from 20 to 28 carbon atoms, comprising:
providing internal monoolefins having of from 10 to 19 carbon atoms;
passing a stream comprising the internal monoolefins having of from 10 to 19 carbon atoms to a disproportionation zone;
operating the disproportionation zone at disproportionation conditions sufficient to disproportionate monoolefins; and
recovering from the disproportionation zone a stream comprising internal monoolefins having of from 20 to 28 carbon atoms.

### Detailed description of the invention

Within the present specification, an average carbon number for a mixture comprising hydrocarbon molecules which differ from each other in terms of carbon number, is determined by multiplying the number of carbon atoms of each hydrocarbon molecule by the weight fraction of that molecule and then adding the products, resulting in a weight average carbon number. The average carbon number may be determined by ¹³C NMR analysis or GC analysis.

Within the present specification, "internal monoolefins having of from 20 to 28 carbon atoms" refers to a mixture comprising internal monoolefin molecules which differ from each other in terms of carbon number. Preferably, said internal monoolefins having of from 20 to 28 carbon atoms have an average carbon number of from 20 to 28, more preferably 21 to 26, more preferably 22 to 25, most preferably 23 to 24.

Within the present specification, "internal monoolefins having of from 10 to 19 carbon atoms" refers to a mixture comprising internal monoolefin molecules which differ from each other in terms of carbon number. Preferably, said internal monoolefins having of from 10 to 19 carbon atoms have an average carbon number of from 10 to 19, more preferably 12 to 19, more preferably 14 to 18, most preferably 16 to 17.

Within the present specification, "internal monoolefins" refers to a mixture comprising monoolefins wherein the proportion of alpha-olefins is below 5 wt.%. Preferably, said mixture of internal olefins comprises less than 3 wt.%, more preferably less than 2 wt.%, most preferably less than 1 wt.% of alpha-olefins. Preferably, the monoolefins are completely isomerized meaning that the double bond is distributed over the carbon chain to the thermodynamic equilibrium.

Further, within the present specification, "internal monoolefins" refers to a mixture which may comprise linear and branched internal monoolefins. Preferably, said mixture of internal monoolefins comprise more than 80 wt.%, more preferably more than 85 wt.%, more preferably more than 90 wt.%, more preferably more than 91 wt.%, most preferably more than 92 wt.% of linear internal monoolefins.

The process of the present invention comprises two steps, the first step being a step wherein internal monoolefins having of from 10 to 19 carbon atoms are provided, and the second step being a disproportionation step. Said first step will be described in more detail hereinafter, followed by the disproportionation step.

In the first step of the process of the present invention, the internal monoolefins having of from 10 to 19 carbon atoms may be provided in any way. The specific process applied to provide such monoolefins is not essential in terms of obtaining the advantages of the present invention.

For example, the internal monoolefins having of from 10 to 19 carbon atoms may be provided by dehydrogenation of the corresponding paraffins also having of from 10 to 19 carbon atoms, which process is described in more detail hereinafter.

Alternatively, such internal monoolefins may be provided by a process comprising first oligomerization of an alkene, preferably ethylene, into alpha-monoolefins having of from 10 to 19 carbon atoms, followed by (double bond) isomerization into the corresponding internal monoolefins also having of from 10 to 19 carbon atoms. Such combination of oligomerization and isomerization may be carried out in a way as disclosed in "Shell's Higher Olefins Process" (SHOP), by Freitas and Gum, CEP, January 1979, pages 73-76, the disclosure of which is incorporated herein by reference. According to said article, only C4, C6, C8, C10 and C20+ alpha-olefins as obtained from ethylene oligomerization, are subjected to isomerization resulting in the corresponding internal olefins. Further, according to said same article, disproportionation of the thus isomerized, internal olefins also results in odd carbon number internal olefins.

In one embodiment of the present invention, the internal monoolefins having of from 10 to 19 carbon atoms are provided by:
passing a stream comprising paraffins having of from 10 to 19 carbon atoms to a dehydrogenation zone;
operating the dehydrogenation zone at dehydrogenation conditions sufficient to dehydrogenate paraffins; and
recovering from the dehydrogenation zone a stream comprising internal monoolefins having of from 10 to 19 carbon atoms.

In such dehydrogenation process, the feed to the dehydrogenation zone is a stream comprising paraffins having of from 10 to 19 carbon atoms. Such paraffin starting material may have been prepared by any process. For example, said stream comprising paraffins having of from 10 to 19 carbon atoms may originate from a Fischer-Tropsch process. The Fischer-Tropsch process is well known to those skilled in the art and can be used for the conversion of synthesis gas (from hydrocarbonaceous feed stocks) into liquid and/or solid hydrocarbons.

In the above-mentioned embodiment, the dehydrogenation of paraffins having of from 10 to 19 carbon atoms may be carried out in any way. The specific process applied to dehydrogenate such paraffins is not essential in terms of obtaining the advantages of the present invention. Suitable dehydrogenation processes are disclosed in EP2186783A2, EP2186784A2 and EP2186785A2, the disclosures of which are incorporated herein by reference.

In the above-mentioned embodiment, the dehydrogenation may be incomplete so that the stream leaving the dehydrogenation zone comprises both internal monoolefins and not-dehydrogenated paraffins, both having of from 10 to 19 carbon atoms. Preferably, the stream leaving the dehydrogenation zone comprises less than 30 wt.%, more preferably less than 20 wt.%, of olefins. In a preferred embodiment of the present invention, the internal monoolefins and not-dehydrogenated paraffins are separated in a separation zone subsequent to dehydrogenation and prior to disproportionation. Thereafter, the separated paraffins may be recycled to the dehydrogenation zone.

Separating said internal monoolefins and not-dehydrogenated paraffins may be carried out in any way. Suitable separation methods are disclosed in EP2186783A2, EP2186784A2 and EP2186785A2, the disclosures of which are incorporated herein by reference. In the above-mentioned embodiment, said internal monoolefins and not-dehydrogenated paraffins are preferably separated by an adsorptive separation process.

The process of the dehydrogenation step in the above-mentioned embodiment of the present invention is not limited to any one particular flow scheme. Said dehydrogenation process may be performed substantially in the manner shown in the scheme of Figure 1 of EP2186783A2, the disclosure of which is incorporated herein by reference.

The disproportionation step of the process of the present invention comprises:
passing a stream comprising the internal monoolefins having of from 10 to 19 carbon atoms to a disproportionation zone;
operating the disproportionation zone at disproportionation conditions sufficient to disproportionate monoolefins; and
recovering from the disproportionation zone a stream comprising internal monoolefins having of from 20 to 28 carbon atoms.

In the disproportionation step, the feed to the disproportionation zone is a stream comprising internal monoolefins having of from 10 to 19 carbon atoms. Reactions of olefins in the presence of a metal-containing catalyst to produce other olefins are known in the art as "disproportionation" or "metathesis" reactions. This olefin disproportionation reaction can be visualized as the breaking of two existing double bonds and the formation of two new double bonds. A typical olefin disproportionation process is one wherein two similar non-symmetrical olefin molecules react in the presence of a metal-containing catalyst to produce one olefin of a higher carbon number and one olefin of a lower carbon number. For example, propylene may be disproportionated to produce ethylene and butylene.

In the present invention, the disproportionation of the internal monoolefins having of from 10 to 19 carbon atoms may be carried out in any way. The specific process applied to disproportionate such olefins is not essential in terms of obtaining the advantages of the present invention. Suitable disproportionation processes are disclosed in US3340322, US3637892, US3760026, US3792108, US3872180, GB1128091, US4568788, US4522936, US45914235, US4629719, US4454368, US3829523, US5254786, US5098876, US5057644 and EP2194034A1, the disclosures of which are incorporated herein by reference.

Thus, a variety of metal-containing catalysts may be employed for conducting the disproportionation reaction in the present invention, such as those disclosed in US3340322, US3637892, US3760026, US3792108, US3872180 and GB1128091, the disclosures of which are incorporated herein by reference. Among the suitable catalysts that have been developed for disproportionation include inorganic refractory materials containing molybdenum and/or tungsten oxide.

Several patents disclose the use of suitable promoter(s) to enhance disproportionation catalyst activity. Elemental metal promoters selected from the group consisting of barium, magnesium, tungsten, silicon, antimony, zinc, manganese and tin are disclosed in US4568788, US4522936, US45914235 and US4629719, the disclosures of which are incorporated herein by reference. In addition, organometallic compounds, such as aluminium and tin alkyls to promote solid catalysts including molybdenum and rhenium oxide for the disproportionation are disclosed in US4454368 and US3829523, the disclosures of which are incorporated herein by reference.

US5254786, US5098876 and US5057644, the disclosures of which are incorporated herein by reference, disclose the use of organoborane compounds as promoters for disproportionation catalysts comprising rhenium and/or molybdenum supported on an inorganic oxide support.

Generally, in the present invention it is preferred that the metal-containing catalyst used in the disproportionation step is a catalyst comprising one or more metals selected from the group consisting of molybdenum (Mo), tungsten (W) and rhenium (Re). More preferably, said catalyst comprises molybdenum. Further, it is preferred that said metal-containing catalyst is a supported catalyst, wherein the support is preferably an inorganic oxide.

The disproportionation step of the present invention can be carried out either batch-wise or continuously, using a fixed catalyst bed, a stirrer equipped reactor or other mobile catalyst contacting process, as well as any other well-known contacting technique. The disproportionation process may be carried out at temperatures ranging from 50 to 300 °C, preferably 100 to 200 °C, and at pressures ranging from 50 to 2000 psig, preferably 100 to 1000 psig. The disproportionation reaction is usually effected in a liquid phase and if desired, liquid reaction diluents are utilized. Examples of suitable diluents are hydrocarbons, such as cyclic or alicyclic alkanes of from 6 to 12 carbon atoms, such as hexane, isooctane and cyclohexane. Also exemplary would be monoaromatic compounds, such as benzene and toluene. If a diluent is added, it may be present in amounts up to 20 moles of diluent per mole of olefinic reactant.

In the present invention, the desired internal monoolefins having of from 20 to 28 carbon atoms are obtained by disproportionating internal monoolefins having of from 10 to 19 carbon atoms. By way of an example, by applying the disproportionation step of the present invention, 4-pentadecene (C15) can be converted into 11-docosene (C22) and 4-octene (C8):

2 C₁₀H₂₁CH=CHC₃H₇ → C₁₀H₂₁CH=CHC₁₀H₂₁ + C₃H₇CH=CHC₃H₇

That is to say, in addition to the desired internal monoolefins having of from 20 to 28 carbon atoms, internal monoolefins having a carbon number outside that range are formed in the disproportionation step of the present invention as well. Said internal monoolefins having of from 20 to 28 carbon atoms, and any other internal monoolefin fraction, may easily be recovered from the product stream leaving the disproportionation zone by any separation technique, including distillation.

As said in the introduction, such C20-C28 internal monoolefins may advantageously be used in the production of internal olefin sulfonates (IOS) which are useful as surfactants in chemical enhanced oil recovery (cEOR). But the other olefin fractions having a carbon number outside that range obtained after disproportionation and separation, may advantageously be used in other applications as well. For example, the C10-C13 monoolefins may be used in the production of linear alkyl benzene sulfonate (LABS) surfactants. Further, the C2-C6 monoolefins may be used in the production of other valuable chemicals, such as ethylene oxide, propylene oxide, etc.

In the present invention, it is preferred that internal monoolefins having of from 10 to 19 carbon atoms, preferably internal monoolefins having of from 14 to 18 carbon atoms, from the product stream leaving the disproportionation zone are recycled to the disproportionation step so that advantageously more of the desired internal monoolefins having of from 20 to 28 carbon atoms can be produced. Alternatively or additionally, other internal monoolefin fractions may be recycled, preferably internal monoolefins having of from 7 to 9 carbon atoms. Preferably, both internal monoolefins having of from 14 to 18 carbon atoms and internal monoolefins having of from 7 to 9 carbon atoms are recycled, so that more of the desired internal monoolefins having of from 20 to 28 carbon atoms can be produced.

The invention is further illustrated by the following Example.

### Example

The carbon number distribution was calculated for the product mixture resulting from disproportionating a mixture comprising internal monoolefin molecules having an average carbon number of 16.5.

Said olefin mixture is a mixture of linear monoolefins having 15, 16, 17 and 18 carbon atoms in equal proportions. That is to say, said mixture comprises 25 wt.% of each of said four C15, C16, C17 and C18 monoolefins. Further, each of said four C15, C16, C17 and C18 monoolefins is completely isomerized meaning that the double bond is distributed over the carbon chain to the thermodynamic equilibrium. This implies that the relative amount of the alpha-olefin is low whereas the relative amount of each of the internal olefins is high. Further, the amount of each internal olefin is the same as the amount of any other internal olefin, except for the internal olefin having an even carbon number and having the double bond at the innermost position (see C16 example below). For example, for the C16 monoolefin the double bond is distributed over the carbon chain to the thermodynamic equilibrium, as follows:

| C16 monoolefin | Amount (mole%) |
|---|---|
| 1-hexadecene | 0.5 |
| 2-hexadecene | 15.3 |
| 3-hexadecene | 15.3 |
| 4-hexadecene | 15.3 |
| 5-hexadecene | 15.3 |
| 6-hexadecene | 15.3 |
| 7-hexadecene | 15.3 |
| 8-hexadecene | 7.7 |
| Total amount (mole%) | 100 |

Further, in the calculation of the carbon number distribution for the product mixture, it was assumed that no double bond isomerization takes place during the disproportionation reaction itself. The calculated carbon number distribution (in wt.%) for the product mixture is shown in the below table for the following 3 sets of conditions:
Set A: no recycle of any fraction to disproportionation;
Set B: recycle of 100 wt.% of the C7-C9 fraction to disproportionation;
Set C: recycle of 100 wt.% of the C7-C9 fraction and 90 wt.% of the C14-C18 fraction to disproportionation.

| | Start | Set A | Set B | Set C |
|---|---|---|---|---|
| 2 | 0 | 0 | 0 | 0 |
| 3 | 0 | 0 | 0 | 0 |
| 4 | 0 | 0.1 | 0.2 | 0.2 |
| 5 | 0 | 0.4 | 0.5 | 0.5 |
| 6 | 0 | 0.6 | 0.9 | 0.9 |
| 7 | 0 | 1.0 | 1.5 | 1.6 |
| 8 | 0 | 1.4 | 2.0 | 2.2 |
| 9 | 0 | 1.8 | 2.5 | 2.7 |
| 10 | 0 | 2.4 | 3.0 | 3.2 |
| 11 | 0 | 3.0 | 3.6 | 3.7 |
| 12 | 0 | 3.6 | 4.2 | 4.4 |
| 13 | 0 | 4.4 | 4.9 | 5.1 |
| 14 | 0 | 5.2 | 5.7 | 6.1 |
| 15 | 25 | 6.2 | 6.7 | 7.0 |
| 16 | 25 | 6.9 | 7.2 | 7.6 |
| 17 | 25 | 7.3 | 7.5 | 7.7 |
| 18 | 25 | 7.4 | 7.4 | 7.5 |
| 19 | 0 | 6.8 | 6.6 | 6.5 |
| 20 | 0 | 6.5 | 6.0 | 5.8 |
| 21 | 0 | 6.1 | 5.4 | 5.2 |
| 22 | 0 | 5.7 | 4.9 | 4.7 |
| 23 | 0 | 5.2 | 4.4 | 4.3 |
| 24 | 0 | 4.6 | 3.8 | 3.8 |
| 25 | 0 | 4.0 | 3.3 | 3.2 |
| 26 | 0 | 3.3 | 2.7 | 2.5 |
| 27 | 0 | 2.5 | 2.1 | 1.7 |
| 28 | 0 | 1.7 | 1.4 | 1.1 |
| 29 | 0 | 1.0 | 0.9 | 0.6 |
| 30 | 0 | 0.5 | 0.4 | 0.3 |
| 31 | 0 | 0.2 | 0.2 | 0.1 |
| 32 | 0 | 0.1 | 0 | 0 |
| 33 | 0 | 0 | 0 | 0 |

From the above table it appears that the proportion of the C19+ internal monoolefins (having carbon number of 19 and higher), which are suitable precursors in the production of internal olefin sulfonates (IOS) which are useful as surfactants in chemical enhanced oil recovery (cEOR), is relatively high:
Set A: 48.3 wt.% of C19+ internal monoolefins;
Set B: 42.1 wt.% of C19+ internal monoolefins; and
Set C: 39.7 wt.% of C19+ internal monoolefins.

Although not recycling to disproportionation, as in "Set A", provides a higher yield of C19+ internal monoolefins (48.3 wt.%), internal recycles are required if no suitable outlet is available for particular carbon cuts, such as said C7-C9 and C14-C18 fractions.

The above calculations were carried out on the basis of 100 t/h of C15-C18 feed. In addition, it has appeared that by also recycling 90 wt.% of the C14-C18 fraction to the disproportionation, as in "Set C", the yield of said C19+ internal monoolefins is advantageously substantially increased from 44.8 t/h (tonnes per hour), as in "Set B", to 64.9 t/h per 100 t/h of C15-C18 feed. In "Set A", the yield of said C19+ internal monoolefins is 48.3 t/h.

## Claims

1. Process for the preparation of internal monoolefins having of from 20 to 28 carbon atoms, comprising:
providing internal monoolefins having of from 10 to 19 carbon atoms;
passing a stream comprising the internal monoolefins having of from 10 to 19 carbon atoms to a disproportionation zone;
operating the disproportionation zone at disproportionation conditions sufficient to disproportionate monoolefins; and
recovering from the disproportionation zone a stream comprising internal monoolefins having of from 20 to 28 carbon atoms.

2. Process according to claim 1, wherein the internal monoolefins having of from 10 to 19 carbon atoms are provided by:
passing a stream comprising paraffins having of from 10 to 19 carbon atoms to a dehydrogenation zone;
operating the dehydrogenation zone at dehydrogenation conditions sufficient to dehydrogenate paraffins; and
recovering from the dehydrogenation zone a stream comprising internal monoolefins having of from 10 to 19 carbon atoms.

3. Process according to claim 2, wherein the stream comprising paraffins having of from 10 to 19 carbon atoms originates from a Fischer-Tropsch process.

4. Process according to claim 2 or 3, wherein the stream leaving the dehydrogenation zone comprises both internal monoolefins and not-dehydrogenated paraffins, both having of from 10 to 19 carbon atoms, and wherein the internal monoolefins and not-dehydrogenated paraffins are separated in a separation zone subsequent to dehydrogenation and prior to disproportionation.

5. Process according to claim 4, wherein the internal monoolefins and not-dehydrogenated paraffins are separated by an adsorptive separation process.

6. Process according to any one of the preceding claims, wherein in the disproportionation a metal-containing catalyst is used which comprises one or more metals selected from the group consisting of molybdenum, tungsten and rhenium.

7. Process according to claim 6, wherein the catalyst comprises molybdenum.

8. Process according to any one of the preceding claims, wherein the disproportionation is carried out at a temperature ranging from 50 to 300 °C.

9. Process according to any one of the preceding claims, wherein the disproportionation is carried out at a pressure ranging from 50 to 2000 psig.

10. Process according to any one of the preceding claims, wherein internal monoolefins having of from 10 to 19 carbon atoms, preferably internal monoolefins having of from 14 to 18 carbon atoms, from the product stream leaving the disproportionation zone are recycled to the disproportionation step.

11. Process according to any one of the preceding claims, wherein internal monoolefins having of from 7 to 9 carbon atoms from the product stream leaving the disproportionation zone are recycled to the disproportionation step.
